Europäisches Patentamt

European Patent Office

Office européen des brevets

(19)

(11) **EP 0 780 117 A1**

(12) **EUROPÄISCHE PATENTANMELDUNG**

(43) Veröffentlichungstag:
25.06.1997 Patentblatt 1997/26

(51) Int. Cl.$^6$: **A61K 7/06**, A61K 7/50

(21) Anmeldenummer: 96116148.6

(22) Anmeldetag: 09.10.1996

(84) Benannte Vertragsstaaten:
**DE ES FR GB IT**

(30) Priorität: **20.12.1995 DE 19547679**

(71) Anmelder: **Wella Aktiengesellschaft**
**64274 Darmstadt (DE)**

(72) Erfinder:
• **Eicken, Ulrich, Dr.**
**1700 Fribourg (CH)**
• **Stiehm, Thomas, Dr.**
**33739 Bielefeld (DE)**

(54) **Haarpflegeemulsion**

(57) Die Erfindung betrifft eine Haarpflegeemulsion enthaltend

a) 0,5 bis 5 Gewichtsprozent mindestens eines Monoglycerids einer gesättigten oder ungesättigten $C_6$-bis $C_{22}$-Fettsäure, eines Polyglycerylmono- oder -polyfettsäureesters einer gesättigten oder ungesättigten $C_6$- bis $C_{22}$-Fettsäure mit 2 bis 10 Glyceryleinheiten an Molekül und/oder eines Esters eines Monosaccharids oder eines Disaccharids mit einer gesättigten oder ungesättigten $C_6$- bis $C_{22}$-Fettsäure,

b) 0,5 bis 5 Gewichtsprozent Trimethylglycin und/oder Panthenol und

c) 0,5 bis 8 Gewichtsprozent mindestens einer gesättigten oder ungesättigten $C_6$- bis $C_{22}$-Fettsäure,
die außer der Komponente a) keine weiteren Tenside enthält.

Die erfindungsgemäße Haarpflegeemulsion enthält ausschließlich Naturstoffe, verbessert die Naß- und Trockenkämmbarkeit sowie den Griff des Haares, verleiht dem Haar einen angenehmen Griff und ein gepflegtes Aussehen.

EP 0 780 117 A1

Printed by Rank Xerox (UK) Business Services
2.14.8/3.4

## Beschreibung

Gegenstand der vorliegenden Erfindung ist eine Haarpflegeemulsion, welche eine Kombination aus Trimethylglycin und/oder Panthenol, mindestens einer $C_6$- bis $C_{22}$-Fettsäure und mindestens einem Monoglycerid einer gesättigten oder ungesättigten $C_6$- bis $C_{22}$- Fettsäure oder einem Polyglycerylmono- oder -polyfettsäureesters einer $C_6$- bis $C_{22}$-Fettsäure mit 2 bis 10 Glyceryleinheiten im Molekül oder einem Ester eines Mono- oder Disaccharids mit einer gesättigten oder ungesättigten $C_6$- bis $C_{22}$-Fettsäure aufweist, das außer den vorgenannten Fettsäureestern keine weiteren Tenside enthält.

Durch öfteres Bleichen, Dauerwellen und Färben, aber auch durch häufiges Waschen der Haare mit entfettenden Tensiden, kommt es zu einer Schädigung der Haarstruktur. Das Haar wird spröde, und es verliert seinen Glanz. Weiterhin lädt sich das Haar beim Kämmen elektrostatisch auf, und die aufgerauhte Haaroberfläche verursacht Verfilzungen sowie Verknotungen des Haares. Hierdurch wird das Kämmen sehr erschwert.

Haarbehandlungsmittel mit einer kämmbarkeitsverbessernden und pflegenden Wirkung haben daher eine erhebliche Bedeutung erlangt. Derartige Mittel werden beispielsweise häufig in Form einer klaren Haarpflegespülung oder auch in Emulsionsform, als sogenannte Creme-Rinses, nach der Haarwäsche im noch nassen Haar verteilt, einige Minuten bis eine Stunde einwirken gelassen und dann mit Wasser ausgespült.

Als Wirkstoffe zur Verbesserung der Haarstruktur werden hauptsächlich synthetische Wirkstoffe, wie zum Beispiel kationische Tenside, kationische Polymere und Fettalkohole eingesetzt.

Die Aufgabe der vorliegenden Erfindung besteht darin, ein Mittel zur Pflege der Haare zur Verfügung zu stellen, das ausschließlich Naturstoffe, das heißt Substanzen pflanzlichen, tierischen oder mineralischen Ursprungs sowie deren Gemische und Reaktionsprodukte untereinander enthält, das lagerstabil ist, die Naß- und Trockenkämmbarkeit sowie den Griff des Haares verleiht und dem Haar einen angenehmen Griff und ein gepflegtes Aussehen verleiht.

Unter Naturstoffen im Sinne der Erfindung werden nur Substanzen verstanden, die durch physikalische Verfahren einschließlich der Extraktion mit Wasser, Ethanol, Glycerin und Kohlensäure oder durch enzymatische oder mikrobiologische Verfahren aus natürlichem Material gewonnen oder weiterverarbeitet werden.

Hierzu wurde nun gefunden, daß eine Haarpflegeemulsion, welche eine Kombination aus

a) 0,5 bis 5 Gewichtsprozent mindestens eines Monoglycerids einer gesättigten oder ungesättigten $C_6$-bis $C_{22}$-Fettsäure, eines Polyglycerylmono- oder - polyfettsäureesters einer gesättigten oder ungesättigten $C_6$- bis $C_{22}$- Fettsäure mit 2 bis 10 Glyceryleinheiten im Molekül und/oder eines Esters eines Monosaccharids oder eines Disaccharids mit einer gesättigten oder ungesättigten $C_6$- bis $C_{22}$- Fettsäure,

b) 0,5 bis 5 Gewichtsprozent Trimethylglycin und/oder Panthenol und

c) 0,5 bis 8 Gewichtsprozent mindestens einer gesättigten oder ungesättigten $C_6$- bis $C_{22}$-Fettsäure,

die mit Ausnahme von Komponente a) frei ist von weiteren Tensiden,
die gestellte Aufgabe in hervorragender Weise löst.

Das erfindungsgemäße Mittel enthält bevorzugt 1 bis 2,5 Gewichtsprozent der Komponente a). Von den als Komponente a) geeigneten Fettsäureestern sind jeweils die Ester der gesättigten $C_{10}$- bis $C_{18}$-Fettsäuren und der Ölsäure, also die Monoglyceride der gesättigten $C_{10}$- bis $C_{16}$- Fettsäuren oder der Ölsäure, die Polyglycerylmono- oder -polyfettsäureester der gesättigten $C_{10}$- bis $C_{18}$- Fettsäuren oder der Ölsäure mit 2 bis 10 Glyceryleinheiten im Molekül und die gesättigten $C_{10}$-bis $C_{18}$- Fettsäureester oder Ölsäureester der Monosaccharide und der Disaccharide bevorzugt.

Bei den gesättigten oder ungesättigten $C_6$- bis $C_{22}$-Fettsäureestern der Mono- oder Disaccharide handelt es sich bevorzugt um $C_6$- bis $C_{22}$-Fettsäureester der Saccharose, Glucose oder Fructose.

Als Komponente a) geeignete Monoglyceride der $C_6$- bis $C_{22}$-Fettsäuren sind beispielsweise das Glycerinmonolaurat, das unter der Handelsbezeichnung Monomuls® 90-L-12, und das Glycerinmonooleat, das unter der Handelsbezeichnung Monomuls® 90-0-18 von der Firma Henkel KGaA, Düsseldorf, BRD, vertrieben wird, zu nennen.

Als Komponente a) geeignete Polyglycerylmono- oder -polyfettsäureester sind beispielsweise die nachstehend genannten, die von der Firma Nikkol Chem. Co. Ltd., Japan unter den in Klammern angegebenen Handelsbezeichnungen vertrieben werden: Diglycerylmonostearat (Nikkol® DGMS), Diglycerylmonooleat (Nikkol® DGMO-C, Nikkol® DGMO-90), Diglyceryldioleat (Nikkol® DGDO), Diglycerylmonoisorstearat (Nikkol® DGMIS), Tetraglycerylmonostearat (Tetraglyn® 1-S), Tetraglycerylmonooleat (Tetraglyn® 1-0), Tetraglyceryltristearat (Tetraglyn® 3-S), Tetraglycerylpentastearat (Tetraglyn® 5-S), Tetraglycerylpentaoleat (Tetraglyn® 5-0), Hexaglycerylmonolaurat (Hexaglyn® 1-L), Hexaglycerylmonomyristat (Hexaglyn® 1-M), Hexaglycerylmonostearat (Hexaglyn® 1-S), Hexaglycerylmonooleat (Hexaglyn® 1-0), Hexaglyceryltristearat (Hexaglyn® 3-S), Hexaglycerylpentastearat (Hexaglyn® 5-S), Hexaglycerylpentaoleat (Hexaglyn® 5-0), Hexaglycerylpolyricinolat (Hexaglyn® PR-15), Decaglycerylmonolaurat (Decaglyn® 1-L), Decaglycerylmonomyristat (Decaglyn® 1-M) und Decaglycerylmonostearat (Decaglyn® 1-S), das von der Firma BASF, Ludwigshafen, BRD, unter der Handelsbezeichnung Cremophor® GO-32 vertriebene Triglyceryldioleat und das unter der Handelsbezeichnung Cremophor GS 32 vertriebene Triglycerylstearat, das unter der Handelsbezeichnung Polyaldo® TGMS von der Firma Lonza Inc, USA vertriebene Triglycerylstearat sowie das unter der Handelsbezeichnung Polyaldo® 10-1-S in Form einer 40prozentigen Lösung von der Lonza Inc. vertriebene Decaglycerylmonostearat.

Das erfindungsgemäße Mittel enthält als Komponente a) besonders bevorzugt einen Ester der Kokosfettsäuren (gesättigte $C_{12}$- bis $C_{14}$-Fettsäuren) der Saccharose, der beispielsweise unter der Handelsbezeichnung Ryoto® LWA 1570 in Form einer 40 prozentigen wäßrig-ethanolischen Lösung von der Firma Ryoto Co. Ltd., Japan vertrieben wird.

Die erfindungsgemäße Haarpflegeemulsion enthält bevorzugt 1 bis 3 Gewichtsprozent der Komponente b), wobei ein Gehalt an einem Gemisch aus Trimethylglycin, für das auch die Bezeichnung Betain gebräuchlich ist, und Panthenol besonders bevorzugt ist.

Die Komponente c) ist in dem Mittel gemäß der vorliegenden Erfindung vorzugsweise in einer Menge von 1,5 bis 3 Gewichtsprozent enthalten. Das erfindungsgemäße Mittel enthält als Komponente c) bevorzugt mindestens eine gesättigte $C_{10}$- bis $C_{18}$- Fettsäure und/oder Ölsäure. Als Komponente c) geeignete gesättigte Fettsäuren sind beispielsweise Myristinsäure oder Kokosfettsäuren.

In einer besonderen Ausführungsform enthält das erfindungsgemäße Mittel zusätzlich zu den Komponenten a), b) und c) 0,005 bis 1 Gewichtsprozent, bevorzugt 0,1 bis 0,3 Gewichtsprozent, Lecithin und/oder 0,1 bis 4 Gewichtsprozent natürliche, kosmetisch verträgliche Öle und/oder 0,1 bis 4 Gewichtsprozent natürliche, kosmetisch verträgliche Wachse.

Ein für die Verwendung im erfindungsgemäßen Mittel geeignetes Lecithin ist Sojalecithin, das beispielsweise von der Firma Laserson S.A., Frankreich, unter der Handelsbezeichnung Mactan® P-97 vertrieben wird und das unter der Handelsbezeichnung Emulmetik® 970 vertriebene Lecithin aus Eiern von der Firma Lucas Meyer, Hamburg, BRD.

Für die Verwendung im erfindungsgemäßen Mittel geeignete, natürliche, kosmetisch verträgliche Öle sind beispielsweise Weizenkeimöl, Sonnenblumenöl, Avocadoöl, Jojobaöl, Babassuöl und Macadamiaöl. Geeignete natürliche, kosmetisch verträgliche Wachse sind beispielsweise Bienenwachs, Apfelwachs, Wollwachs, Flachswachs und Shea Butter.

Die erfindungsgemäße Haarpflegeemulsion kann darüber hinaus alle diejenigen Zusatzstoffe enthalten, die üblicherweise in Mitteln zur Pflege der Haare eingesetzt werden und die Naturstoffe im Sinne der vorgenannten Definition sind, insbesondere natürliche organische oder anorganische Verdickungsmittel, wie zum Beispiel Xanthan Gummi oder ein Gemisch aus Natriumalginat, Glucose, Guar Gummi und Carageenan, das von der Firma Danisco Århus/DK unter der Handelsbezeichnung Cremodan® DC vertrieben wird, in einer Menge von 0,5 bis 10 Gewichtsprozent; organische Säuren, wie zum Beispiel Zitronensäure oder Milchsäure; Konservierungsmittel wie Sorbinsäure, Salicylsäure, Benzoesäure, Propionsäure, 4-Hydroxybenzoesäure und Ameisensäure und ihre Salze, 2-Phenoxyethanol und Benzylalkohol, in einer Menge von 0,05 bis 1 Gewichtsprozent, Antioxidantien, wie zum Beispiel antioxidierend wirkende Pflanzenextrakte, wie Rosmarinextrakt, der beispielsweise von der Firma RAPS unter der Handelsbezeichnung Stabiliton® OS vertrieben wird, in einer Menge von 0,1 bis 1,5 Gewichtsprozent, Feuchthaltemittel wie zum Beispiel Glycerin, Saccharide und Hyaluronsäure, in einer Menge von 0,1 bis 2 Gewichtsprozent; Pflegestoffe, wie zum Beispiel Lanolin, Cholesterin, Proteinhydrolysate, wie zum Beispiel Keratinhydrolysat, und Panthotensäure, in einer Menge von 0,1 bis 10 Gewichtsprozent und natürliche Parfümöle, soweit solche Zusätze nützlich und zweckmäßig erscheinen und mit den Bestandteilen des erfindungsgemäßen Mittels verträglich sind.

Das erfindungsgemäße Mittel enthält außer der Komponente a) keine weiteren nicht-ionischen Tenside und ist frei von amphoteren, anionischen und kationischen Tensiden.

Das erfindungsgemäße Mittel liegt in Form einer Öl-in-Wasser-Emulsion vor und weist einen Wassergehalt von 60 bis 98 Gewichtsprozent, bevorzugt von 80 bis 95 Gewichtsprozent und einen pH-Wert von 2 bis 7, bevorzugt von 3 bis 5, auf.

In einer besonderen Ausführungsform der erfindungsgemäßen Haarpflegeemulsion enthält diese zur gleichzeitigen Tönung des Haares 0,05 bis 2,0 Gewichtsprozent mindestens eines natürlichen Farbstoffs, wie zum Beispiel Pflanzenfarbstoffe wie Henna oder Reng oder Anthocyanidfarbstoffe.

Die Haarpflegeemulsion gemäß der vorliegenden Erfindung wird, üblicherweise nach der Haarwäsche, im handtuchtrockenen Haar je nach Haarfülle in einer Menge von 5 bis 20 g verteilt. Nach einer Einwirkungszeit von 1 bis 15 Minuten wird das Haar mit Wasser ausgespült und sodann getrocknet.

Die Einwirkungszeit des erfindungsgemäßen Mittels hängt innerhalb der gegebenen zeitlichen Grenzen von dem Verwendungszweck des Mittels ab. Handelt es sich um eine Spülung, so beläßt man sie bei 1 bis 5 Minuten auf dem Haar, während bei einer Haarkur eine Einwirkungsdauer von 3 bis 15 Minuten üblich ist.

Die nachstehenden Beispiele sollen den Gegenstand der Erfindung näher erläutern.

**Beispiele**

Beispiel 1: Naturkosmetik-Haarspülung

| | |
|---|---|
| 2,70 g | Kokosfettsäuresaccharoseester (Ryoto® LWA 1570 der Firma Ryoto Co. Ltd.) |
| 1,00 g | Panthenol |
| 1,00 g | Trimethylglycin |
| 1,50 g | Myristinsäure |
| 0,20 g | Sojalecithin (Mactan® P-97) |

| | |
|---|---|
| 3,50 g | Weizenkeimöl |
| 1,50 g | Bienenwachs |
| 0,20 g | Benzoesäure |
| 0,50 g | natürliches Parfümöl |
| 0,50 g | Xanthan Gummi |
| 87,40 g | Wasser |
| 100,00 g | |

Beispiel 2: Haarpflegeemulsion

| | |
|---|---|
| 1,00 g | Saccharoselaurat |
| 2,00 g | Panthenol |
| 1,50 g | Myristinsäure |
| 4,00 g | Weizenkeimöl |
| 1,00 g | Apfelwachs |
| 0,10 g | Sojabohnenlecithin |
| 0,15 g | Natriumbenzoat |
| 0,23 g | Natriumformiat |
| 0,50 g | natürliche Parfümöle |
| 0,50 g | Xanthan Gummi |
| 0,85 g | Zitronensäure |
| 0,10 g | Ethanol |
| 88,07 g | Wasser |
| 100,00 g | |

Beispiel 3: Haarpflegeemulsion

| | |
|---|---|
| 1,00 g | Saccharoselaurat |
| 2,00 g | Trimethylglycin |
| 1,50 g | Myristinsäure |
| 4,00 g | Weizenkeimöl |
| 1,00 g | Apfelwachs |
| 0,10 g | Sojabohnenlecithin |
| 0,15 g | Natriumbenzoat |
| 0,23 g | Natriumformiat |
| 0,50 g | natürliche Parfümöle |
| 0,50 g | Xanthan Gummi |
| 0,85 g | Zitronensäure |
| 0,10 g | Ethanol |
| 88,07 g | Wasser |
| 100,00 g | |

Beispiel 4: Haarpflegeemulsion

| | |
|---|---|
| 1,002 g | Saccharoselaurat |
| 2,000 g | Panthenol |
| 2,000 g | Trimethylglycin |
| 0,500 g | Palmitinsäure |
| 0,500 g | Stearinsäure |
| 1,000 g | Bienenwachs |
| 3,000 g | Mandelöl |
| 2,000 g | Trimethylglycin |
| 0,150 g | Natriumbenzoat |
| 0,230 g | Natriumformiat |
| 0,500 g | natürliche Parfümöle |
| 0,500 g | Xanthan Gummi |
| 0,300 g | Zitronensäure |
| 0,100 g | Ethanol |
| 88,218 g | Wasser |

| | |
|---|---|
| 100,00 g | |

Beispiel 5: Haarpflegeemulsion

| | |
|---|---|
| 0,100 g | Triglycerylmonostearat |
| 0,873 g | Decaglycerylmonostearat |
| 2,000 g | D-Panthenol |
| 2,000 g | Trimethylglycin |
| 1,500 g | Palmitinsäure |
| 1,500 g | Stearinsäure |
| 2,000 g | Mandelöl |
| 1,000 g | Bienenwachs |
| 0,500 g | Teebaumöl |
| 0,500 g | natürliches Parfümöl |
| 0,200 g | Rosmarinextrakt |
| 0,500 g | Xanthan Gummi |
| 0,027 g | Polyglycerin |
| 0,300 g | Zitronensäure |
| 87,000 g | Wasser |
| 100,00 g | |

Beispiel 6: Haarpflegeemulsion

| | |
|---|---|
| 0,100 g | Triglycerylmonostearat |
| 0,873 g | Decaglycerylmonostearat |
| 2,000 g | D-Panthenol |
| 2,000 g | Trimethylglycin |
| 1,500 g | Palmitinsäure |
| 1,500 g | Stearinsäure |
| 2,000 g | Mandelöl |
| 1,000 g | Bienenwachs |
| 0,150 g | Natriumbenzoat |
| 0,230 g | Natriumformiat |
| 0,500 g | natürliche Parfümöle |
| 0,300 g | Zitronensäure |
| 0,500 g | Xanthan Gummi |
| 0,027 g | Polyglycerin |
| 87,320 g | Wasser |
| 100,000 g | |

Alle in der vorliegenden Anmeldung angegebenen Prozentzahlen stellen, sofern nicht anders vermerkt, Gewichtsprozent dar.

**Patentansprüche**

1. Haarpflegeemulsion enthaltend

a) 0,5 bis 5 Gewichtsprozent mindestens eines Monoglycerids einer gesättigten oder ungesättigten $C_6$- bis $C_{22}$-Fettsäure, eines Polyglycerylmono- oder -polyfettsäureesters einer gesättigten oder ungesättigten $C_6$- bis $C_{22}$-Fettsäure mit 2 bis 10 Glycerineinheiten im Molekül und/oder eines Esters eines Monosaccharids oder eines Disaccharids mit einer gesättigten oder ungesättigten $C_6$- bis $C_{22}$-Fettsäure,

b) 0,5 bis 5 Gewichtsprozent Trimethylglycin und/oder Panthenol und

c) 0,5 bis 8 Gewichtsprozent mindestens einer gesättigten oder ungesättigten $C_6$- bis $C_{22}$-Fettsäure,

die außer der Komponente a) keine weiteren Tenside enthält.

2. Mittel nach Anspruch 1, dadurch gekennzeichnet, daß es 0,005 bis 1 Gewichtsprozent Lecithin enthält.

3. Mittel nach einem der Ansprüche 1 oder 2, dadurch gekennzeichnet, daß es 0,1 bis 4 Gewichtsprozent natürliche, kosmetisch verträgliche Öle enthält.

4. Mittel nach einem der Ansprüche 1 bis 3, dadurch gekennzeichnet, daß es 0,1 bis 4 Gewichtsprozent natürliche, kosmetisch verträgliche Wachse enthält.

5. Mittel nach einem der Ansprüche 1 bis 4, dadurch gekennzeichnet, daß es als Komponente a) ein Monglycerid oder einen Polyglycerylmono- oder -polyfettsäureester, mit 2 bis 10 Glyceryleinheiten im Molekül, einer gesättigten $C_{10}$- bis $C_{18}$-Fettsäure oder der Ölsäure, oder einen Ester eines Monosaccharids oder eines Disaccharids mit einer gesättigten $C_{10}$- bis $C_{18}$-Fettsäure oder mit Ölsäure oder deren Gemisch enthält.

6. Mittel nach einem der Ansprüche 1 bis 5, dadurch gekennzeichnet, daß es als Komponente a) einen Ester einer gesättigten $C_{12}$- bis $C_{14}$-Fettsäure mit Saccharose enthält.

**Europäisches Patentamt**

# EUROPÄISCHER RECHERCHENBERICHT

Nummer der Anmeldung

EP 96 11 6148

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | PATENT ABSTRACTS OF JAPAN vol. 95, no. 006, 31.Juli 1995 & JP 07 069840 A (KAO CORP), 14.März 1995, * Zusammenfassung * --- | 1,4,6 | A61K7/06 A61K7/50 |
| A | WO 93 24101 A (PROCTER & GAMBLE ;BRIGGS GILLIAN SCOTT (GB); DATE ROBERT FRANCIS ()) 9.Dezember 1993 * Anspruch 1; Beispiele * * Seite 10, Zeile 3 - Zeile 15 * --- | 1 | |
| A | US 4 737 360 A (ALLEN DAVID W ET AL) 12.April 1988 * Beispiele 2,4,6 * --- | 1,3-5 | |
| A | EP 0 541 830 A (ISP VAN DYK INC) 19.Mai 1993 --- | | |
| A | WO 92 07543 A (HENKEL KGAA) 14.Mai 1992 ----- | | |

**RECHERCHIERTE SACHGEBIETE (Int.Cl.6)**

A61K

Der vorliegende Recherchenbericht wurde für alle Patentansprüche erstellt

| Recherchenort | Abschlußdatum der Recherche | Prüfer |
|---|---|---|
| BERLIN | 25.März 1997 | McConnell, C |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
...............................................................
& : Mitglied der gleichen Patentfamilie, übereinstimmendes Dokument

EPO FORM 1503 03.82 (P04C03)